(19) Europäisches Patentamt
European Patent Office
Office européen des brevets

(11) **EP 3 501 599 A1**

(12) **EUROPÄISCHE PATENTANMELDUNG**

(43) Veröffentlichungstag:
**26.06.2019 Patentblatt 2019/26**

(21) Anmeldenummer: **17208438.6**

(22) Anmeldetag: **19.12.2017**

(51) Int Cl.:
*A61N 1/40* *(2006.01)*   *A61M 37/00* *(2006.01)*
*A61N 1/36* *(2006.01)*   *A61N 2/00* *(2006.01)*
*A61N 2/02* *(2006.01)*   *A61M 21/00* *(2006.01)*

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD TN**

(71) Anmelder: **BEMER Int. AG**
**9495 Triesen (LI)**

(72) Erfinder: **KLOPP, Rainer-Christian**
**16321 Bernau (DE)**

(74) Vertreter: **Gulde & Partner**
**Patent- und Rechtsanwaltskanzlei mbB**
**Wallstraße 58/59**
**10179 Berlin (DE)**

(54) **VORRICHTUNG ZUR STIMULIERUNG DER LOKALEN REGULATION DER MIKROZIRKULATION, PROPHYLAKTISCHES ODER THERAPEUTISCHES VERFAHREN SOWIE VERWENDUNG ZUR VERBESSERUNG REPRÄSENTATIVER MERKMALE DES FUNKTIONSZUSTANDES DER MIKROZIRKULATION**

(57)   Die Erfindung betrifft eine Vorrichtung zur Stimulierung der lokalen Regulation der Mikrozirkulation zur Erzeugung eines biorhythmisch definierten elektromagnetischen Feldes mit unterschiedlichen Impulsfolgen, Frequenzwerten und Magnetfeldstärken, wobei die Vorrichtung einen separaten Schallgeber für ein elastomechanisches Signal zur Erzeugung eines elastomechanischen Feldes aufweist mit definierten Schall-Leistungen, Schallstärken und Schallwechseldrücken. Über eine Messwerterfassungeinrichtung und ein Steuergerät werden die Signale variiert. Die Kombination elektromagnetisches Feld und elastomechanisches Feld führt zu Änderungen repräsentativer Merkmale des Funktionszustandes der Mikrozirkulation des Blutes, die die Summe aus den Einzelwirkungen beider angewendeter Felder bei weitem übersteigt.

EP 3 501 599 A1

**Beschreibung**

Die Erfindung betrifft eine Vorrichtung zur Stimulierung der lokalen Regulation der Mikrozirkulation sowie ein prophylaktisches oder therapeutisches Stimulierungsverfahren und die Verwendung zur Verbesserung repräsentativer Merkmale des Funktionszustandes der Mikrozirkulation.

Es ist in den medizinischen Wissenschaften allgemein anerkannt, dass die Mikrozirkulation der funktionell wichtigste Teil des menschlichen Blutkreislaufes ist, da hier der lebensnotwendige Stoffaustausch mit den Körperzellen stattfindet und zugleich in dieser Region die ersten Schritte immunologischer Reaktionen ablaufen. Es ist ferner allgemein anerkannt, dass eine große Reihe von Erkrankungen durch Störungen der Mikrozirkulation verursacht wird oder zumindest ein Krankheitsgeschehen mit zunehmender eigener Dynamik begleiten. Die derzeit zur Verfügung stehenden (medikamentös-)therapeutischen Optionen sind sehr limitiert. Es hat daher nicht an Versuchen gefehlt bei einer Behandlung der gestörten Mikrozirkulation anstelle oder ergänzend einer Übertragung chemischer Energie (Medikamente) auch bestimmte Möglichkeiten einer Übertragung physikalischer Energie in Betracht zu ziehen.

So sind beispielsweise Methoden der Musiktherapie mit begrenzten Wirkungen eingeführt worden (Klangschalen-Anwendung, Klangsteine u.a.). Einige wenige Untersuchungen liefern hierzu Versuche wissenschaftlicher Begründungen, wobei keine ausreichenden Analysen zu den Schallspektren (Klangspektren) vorliegen.

Ein weiteres Einsatzgebiet betrifft die Anwendung bestimmter elektromagnetischer Felder als Stimulation (physiologischer Reiz) der schubspannungsabhängigen endothelvermittelten Tonusregulation der Arteriolen, des nach heutigem Kenntnisstand im Fachgebiet wichtigstem lokalen Regulationsmechanismus der Organdurchblutung. Hierzu liegen eine Anzahl wissenschaftlicher Publikationen in anerkannten Fachzeitschriften vor, in denen sowohl theoretische Erläuterungen gegeben werden als auch Ergebnisse klinischer Studien zu erfolgreichen Anwendungen mitgeteilt werden. Jedoch sind die Beträge der nachgewiesenen Merkmaländerungen untersuchter mikrozirkulatorischer Funktionsmerkmale nicht ausreichend, um causal-therapeutisch wirksam sein zu können, so dass oftmals eine adjuvante oder additive Anwendung empfohlen werden kann, bevorzugt zur Prophylaxe und als ergänzende Therapiemaßnahme.

Die EP 0 995483 beschreibt eine Vorrichtung, mit der durch pulsierende elektromagnetische Felder die Sauerstoffutilisation und Stoffwechselvorgänge beeinflusst werden sollen.

In der WO 2008/025731 wird eine Vorrichtung zur Erzeugung eines gepulsten elektromagnetischen Feldes mit bestimmten periodischen Impulsen in Abhängigkeit von Messdaten der Mikrozirkulation des Blutes beschrieben.

Die WO 2011/023635 betrifft ebenfalls eine Vorrichtung zur Erzeugung eines gepulsten elektromagnetischen Feldes, wobei genaue Impulsfolgen und Frequenzfolgen beansprucht werden.

Die DE 198 34 148 A1 beschreibt ein Verfahren zur gepulsten Magnetfeldabgabe mit einer Modulation der Amplitude und/oder Frequenz der erzeugten Magnetfelder entsprechend einem elektromagnetischen Signal einer Tonwiedergabequelle.

Der Erfindung liegt die Aufgabe zugrunde, eine Vorrichtung bereitzustellen, mit der die lokale Regulierung im Bereich der Mikrozirkulation des Blutes durch zusätzliche Einrichtungen neben den Einrichtungen zur Erzeugung eines gepulsten elektromagnetischen Feldes signifikant verbessert wird. Eine weitere Aufgabe besteht darin, eine individuelle Anpassung der Signalreize zu ermöglichen und dadurch den Behandlungserfolg zu optimieren.

Eine weitere Aufgabe besteht in der Bereitstellung eines prophylaktischen und therapeutischen Verfahrens zur Stimulierung der lokalen Regulation der Mikrozirkulation mit therapierelevanten Ergebnissen sowie die Verwendung einer Vorrichtung zur Erzeugung eines biorhythmisch definierten Feldes.

Erfindungsgemäß ist die Vorrichtung zur Stimulierung der lokalen Regulation der Mikrozirkulation bei Säugern unter Einbeziehung eines Impulsgenerators (2a) und einer Felderzeugungseinrichtung (2b) zur Erzeugung eines biorhythmisch definierten elektromagnetischen Feldes mit unterschiedlichen Impulsfolgen, Frequenzwerten und Magnetfeldstärken sowie einer Messwerterfassungseinrichtung, enthaltend Geräte zur Intravitalmikroskopie (3) und kombinierten Weißlichtspektroskopie und Laser-Doppler-Mikroflussmessung (4),
dadurch gekennzeichnet, dass die Vorrichtung einen separaten Schallgeber (1) für ein elastomechanisches Signal zur Erzeugung eines biorhythmisch definierten elastomechanischen Feldes aufweist, wobei der Schallgeber (1) Schall-Leistungen im Bereich von $10^{-5}$ W bis 100 W, Schallstärken im Bereich von $10^{-12}$ W/m$^2$ bis 1 W/m$^2$ und Schallwechseldrücke im Bereich von $0,2 \cdot 10^{-4}$ N/m$^2$ bis $10^3$ N/m$^2$ hat,
und ein Steuergerät (5) zur Koordinierung eingehender Messwerte der Messwerterfassungseinrichtung und ausgehender Steuerimpulse auf den Impulsgenerator (2a), die Felderzeugungseinrichtung (2b) und den Schallgeber (1).

Durch experimentelle Untersuchungen wurde überraschend gefunden, was nach bisherigem Kenntnisstand im Fachgebiet nicht erklärbar ist, dass bei einer Kombination der beiden Reize - elektromagnetisches Feld und elastomechanisches Feld - Merkmaländerungen repräsentativer Merkmale des Funktionszustandes der Mikrozirkulation erzielt werden, welche die Summe aus den Einzelwirkungen beider angewendeter Felder bei weitem übersteigt. Die Kombination aus beiden Reizen bewirkt deutlich mehr als die Summe der beiden Teilreize.

Dies zeigt sich beispielhaft bei der venolenseitigen Sauerstoffausschöpfung $\Delta pO_2$ und beim Strömungsfluss der roten Blutzellen $Q_{RBC}$, ist aber auch bei anderen mikrozirkulatorischen Funktionsmerkmalen wie der Anzahl der blutzellperfundierten Knotenpunkte nNP als Maß für den Verteilungszustand des Plasma-Blutzell-Gemisches in den Mikrogefäßen oder dem arteriolären oder venulären Vasomotionszustand AVM zu beobachten. Besonders eindrucksvoll bei diesen Merkmalen sind die langen Abklingquoten der gemessenen Werte. Bei wiederholten Behandlungen wie z.B. täglich oder mehrmals täglich führt das zu causaltherapeutischen Wirkungen auf den Funktionszustand der Mikrozirkulation bis zu einer Verdoppelung der Abklingzeiten der Merkmalsänderungen. Damit ist der Weg zu einem therapeutischen Verfahren eröffnet, das eine weitgehend kontinuierliche Verbesserung des mikrozirkulatorischen Funktionszustandes ermöglicht, wie dies beispielsweise bei nahezu allen sogenannten peripheren Durchblutungsstörungen von Bedeutung ist, insbesondere bei Diabetes Typ II.

Die lokale Regulation der Organdurchblutung erfolgt neben der nervalen und humoralen Ansteuerung der Durchmesser der großkalibrigen Arteriolen vornehmlich über die schubspannungsabhängige endothelvermittelte Tonusregulation der den Kapillaren vorgeschalteten kleinkalibrigen Arteriolenverzweigungen, wobei diese vasomotorischen Vorgänge bestimmten Periodizitäten - Biorhythmen - unterworfen sind, welche im Stör- oder Krankheitsfall Restriktionen und Reduktionen unterliegen.

Ein biorhythmisch definiertes elektromagnetisches Feld, welches z.B. im Krankheitsfall als physikalischer oder physiologischer Reiz Anwendung findet, ist daher die Simulation der naturgegebenen normalen physiologischen Vasomotions-Periodizitäten. Bei der Übertragung elektromagnetischer Feldenergie erfolgt dies daher immer mittels eines bestimmten elektromagnetischen Wechselfeldes. In gleicher Weise gilt dies auch für ein biorhythmisch definiertes elastomechanisches Feld. Ein statisches magnetisches oder elektromagnetisches Feld stellt daher keinen wirksamen physiologischen Stimulationsreiz für die lokale Durchblutungregulation dar.

Das angewandte elastomechanische Signal stellt eine zusammengesetzte Schwingung dar, die sich aus sinusförmigen Teilschwingungen zusammensetzt. Ein für die Erfindung bevorzugtes elastomechnisches Signal weist sinusförmige Teilschwingungen mit Frequenzen vor allem im Bereich von 6 bis 12 Hz und 26 bis 36 Hz auf. Dies wurde empirisch gefunden.

Empirisch wurde ferner ermittelt, dass die zusammengesetzte Schwingung, welche sich als Welle im Raum, hier: im Gewebe, ausbreitet, etwa 1 bis 5 Wellenberge und zugehörige Wellentäler pro Minute aufweisen sollte, vorzugsweise im Mittel 3 pro Minute, bezogen auf die Schwingungsweiten T, welche die zeitliche Distanz zwischen zwei Schwingungszuständen in gleicher Schwingungsphase bezeichnen. Bei Wellenvorgängen gilt entsprechendes für die räumliche Distanz. Dabei gehorchen die Flanken der Wellenberge/-täler bevorzugt parabolischen oder anderen exponentiellen Funktionen. Gemeint ist hier eine idealisierte Mittelwertkurve der zusammengesetzten Schwingung. Die Halbwertsbreiten liegen dann bei etwa 5 bis 10 s.

Als Schallgeber finden alle Verfahren Anwendung, welche durch Energien aller Formen angeregt werden können (Umwandlungen in mechanische Schwingungsenergie), beispielsweise durch elektromechanische Wandler.

Die Schwingungsenergie ist bevorzugt möglich durch Kontakt des Schallgebers mit der Hautoberfläche, beispielweise durch Membranen, schwingungsfähige Platten, sogenannte Schallköpfe und ähnliche. Einsetzbar ist auch ein elektromechanischer Wandler (elektromagnetische oder elektrodynamische Wechselwirkungen) oder ein Thermophon. Zur Verbesserung der Übertragung der elektromechanischen Schwingungsenergie durch den Schallgeber kann ein akustisch dichtes Kopplungsmedium geringer Schichtdicke eingesetzt werden, z.B. wie es in der Ultraschalldiagnostik Anwendung findet.

Membranen und/oder schwingungsfähige Platten sind bevorzugt.

Targetstrukturen der gesetzten Reize sind vornehmlich die mikrovaskulären Netzwerke insbesondere in oberflächennahen Geweben wie Subkutis oder Skelettmuskulatur.

Für die Übertragung des elastomechanischen Signals, d.h. die spezifische Schallwelle, auf ein Gewebe gelten die Gleichungssysteme der Elastomechanik.

Ein Schallfeld wird durch die Größen Schallschnelle und Schalldruck vollständig beschrieben.

Leitmerkmal für die medizinische Anwendung der Vorrichtung ist der Schalldruck p (Schallwechseldruck), welcher vom Schallstrahlungsdruck zu unterscheiden ist.

Der Schalldruck p, eine Kraft pro Fläche, ist der als Folge der Schwingungen der Gewebeteilchen, welche das Schallausbreitungsmedium darstellen, resultierende Wechseldruck.

Es gilt

$$p = P\cos 2\pi v\left(t - x/c_s\right)$$

Hierin bedeuten

$v$ ist die Frequenz einer ebenen, sich in x-Richtung mit der Geschwindigkeit $c_s$ ausbreitenden elastomechanischen

Welle.

P ist die Schallwechseldruck-Amplitude.

$$P = 2\pi\rho c_s A = \rho c_s U$$

mit A der Schwingungsamplitude, U der Geschwindigkeitsamplitude und $\rho$ der Dichte des Ausbreitungsmediums.

Gleichzeitig gilt für P

$$P = \sqrt{2\rho c_s J}\,,$$

wobei J die Schallintensität ist.

Man beachte, dass diese Beziehungen nur für kleinere Werte von A, U und P gelten. Im Fall von starken Schallfeldern tritt in der Beziehung für den Schallwechseldruck p zum linearen Term ein quadratischer Term, welcher den Schallstrahlungsdruck als Funktion der mittleren

Energiedichte $\overline{E}$

darstellt. Die Beziehung für den Schallwechseldruck lautet dann

$$p = P\cos 2\pi v\left(t - x/c_s\right) + 2\overline{E}\cdot\cos^2 2\pi v\left(t - x/c_s\right).$$

Für die Vorrichtung sind Schallwechseldrücke im Bereich von $0{,}2 \cdot 10^{-4}$ N/m$^2$ bis $10^3$ N/m$^2$ geeignet. Die Schallschnelle $v$ bezeichnet die Geschwindigkeit schwingender Teilchen des Ausbreitungsmediums einer elastomechanischen Welle. Für harmonische Schallwellen gilt die Beziehung

$$v = \omega A$$

zwischen der Schallschnelle $v$, der Kreisfrequenz $\omega = 2\pi v$ (v Frequenz) und der Schwingungsamplitude A der schwindenden Teilchen im Schallfeld.

Durch die Angabe von p und $v$ wird ein Schallfeld charakterisiert. Wenn das Schallausbreitungsmedium die Schallwelle absorbiert, dann tritt zwischen beiden Schallfeld-Größen eine von Null verschiedene Phasenverschiebung $\phi$ auf. Der durch das Verhältnis aus Schalldruck p und Schallschnelle $v$ definierte Schallwellenwiderstand R = p/$v$ (OHM'sches Gesetz der Schallschnelle) ist somit meist komplex. In stehenden Schallwellen ist $\phi = 90°$.

Die Schall-Leistung Ps ist die in der Zeiteinheit durch eine die Schallquelle umschließende Fläche F strömende Schallenergie, welche durch Integration der Schallintensität J über diese Fläche erhalten wird:

$$P_s = \oiint_F J\,df\,.$$

Zu berücksichtigen sind Schall-Leistungen im Bereich von $10^{-5}$ W bis ca. 100 W. Diese können unter Umständen bis zu 1 kW erhöht werden.

Die Schallstärke oder Schallintensität J ist die in der Zeiteinheit 1s durch die senkrecht zur Schallausbreitungsrichtung stehende Einheitsfläche 1m$^2$ strömende Schallenergie W.

Aus den Schallfeld-Größen Schalldruck p und Schallschnelle $v$ berechnet sich J

$$J = p_{eff} \, v_{eff} \, \cos \phi$$

,

wobei der Index eff die Benutzung der Effektivwerte von p und *v* vorschreibt. $\phi$ ist die Phasenverschiebung zwischen p und *v*.

Für die Vorrichtung vorteilhafte Schallstärken liegen im Bereich von $10^{-12}$ W/m$^2$ bis 1 W/m$^2$

Das anzuwendende elastomechanische Signal stellt eine zusammengesetzte Schwingung dar, welche sich durch FOURIER-Synthese aus sinusförmigen Teilschwingungen zusammensetzt. Aus Resultaten empirischer Untersuchungen ergab sich, dass sich hierzu nicht ausschließlich, jedoch bevorzugt Teilschwingungen mit Frequenzen von ca. 6 - 12 Hz und 26 - 36 Hz eignen.

Für die Frequenzregulation des anzuwendenden elastomechanischen Feldes wird bei elektromechanischer Kopplung auf die bekannten Einrichtungen der Elektroakustik zurückgegriffen, wie Hochpassfilter und Tiefpassfilter mit entsprechend in Reihe geschalteter Widerstände (OHM'scher Widerstand, kapazitiver Widerstand, induktiver Widerstand).

Das eingestrahlte Schallfeld erfährt im Gewebe (unterschiedliche Gewebe mit unterschiedlichen Grenzflächen, unterschiedliche Elastizitätskoeffizienten) eine Reihe von Wechselwirkungen: Streuung, Beugung, Brechung, Reflexion und Absorption. Da die Schalldurchlässigkeit mit wachsender Frequenz abnimmt, weil die Schallabsorption mit steigender Frequenz zunimmt, ist die Anwendung geringer Schallfrequenzen für eine möglichst optimale Durchdringung des Schallfeldes der verschiedenen histologischen Strukturen von Vorteil.

Bei der Festlegung des therapierelevanten elastomechanischen Signals ist das Schallspektrum von Bedeutung (Amplituden-Frequenz-Diagramm). Die frequenzabhängigen Amplituden der Schallintensität des angewendeten Schallfeldes können durch proportionale Schalldruck-Amplituden vertreten werden. Das davon zu unterscheidende Klangspektrum stellt eine Darstellung nach FOURIER des in Teiltöne zerlegten Klanges in einem Frequenz-Amplituden-Diagramm mittels Klanganalyse dar und ist ein Sonderfall des allgemeinen Schallspektrums. Das Schallspektrum kann eine Vielzahl in allen möglichen Verhältnissen zueinander stehenden Frequenzen enthalten, während ein Klangspektrum immer nur Frequenzen enthält, die ein ganzzahliges Vielfaches des Grundtones darstellen.

In dem biorhythmisch definierten Schallfeld können sowohl das Schallspektrum als auch das Klangspektrum bei der Definition des Reizsignals Anwendung finden.

Bevorzugte Ausführungsformen der Erfindung bestehen darin, dass die Flussdichte des elektromagnetischen Feldes im Bereich von 5 $\mu$T bis 0,1 T liegt, vorzugsweise im Bereich von 150 $\mu$T bis 90 mT und insbesondere im Bereich: von 400 $\mu$T bis 60 mT.

Eine weitere bevorzugte Ausführungsform besteht darin, dass die Frequenz des elektromagnetischen Feldes im Bereich von 6 bis 40 Hz liegt.

Besonders bevorzugte Impulsfolgen für das elektromagnetische Feld sind in den Veröffentlichungen WO2011/023634 und WO2011/023635 beschrieben.

Eine weiter bevorzugte Ausführungsform besteht darin, dass der Bereich für den Schallwechseldruck bei 0,5 • $10^{-3}$ N/m$^2$ bis 10$^2$ N/m$^2$ liegt, vorzugsweise 0,5 • $10^{-2}$ N/m$^2$ bis 1 N/m$^2$, insbesondere 0,5 • $10^{-2}$ N/m$^2$ bis 0,5 N/m$^2$. Die Schall-Leistung liegt vorzugsweise bei $10^{-3}$ W bis 8 W, insbesondere $10^{-2}$ W bis 0,5 W. Die Schallstärke liegt vorzugsweise bei $10^{-10}$ W/m$^2$ bis 0,1 W/m$^2$, insbesondere $10^{-6}$ W/m$^2$ bis $10^{-2}$ W/m$^2$.

Die Einwirkung des gepulsten elektromagnetischen Feldes kann über eine Matte erfolgen, in der einzelne elektromagnetische Spulen in der Gesamtfläche verteilt sind. Damit ist eine Ganzkörperapplikation möglich. Die Einwirkung kann auch über ein kleineres Pad von ca. 8-10 cm Durchmesser zur gezielten Anregung kleinerer Gewebe-oder Muskulaturbereiche des Patienten erfolgen als lokale Anwendung, zeitgleich mit einem elastomechanischen Signal in der gleichen Körperregion.

Bei der erfindungsgemäßen Vorrichtung kommt dem Steuergerät 5 eine zentrale Aufgabe zu. So muss von dem Steuergerät 5 bei eingehenden Messwerten der Erfassungseinrichtung 3;4 in der Weise in Form von Steuerimpulsen reagiert werden, dass z.B. der Frequenzbereich des Schallgebers 1 erhöht oder abgesenkt wird und/oder der Schallwechseldruck verringert oder stufenweise erhöht wird oder die magnetische Flussdichte B des elektromagnetischen Feldes, die Impulsfolge oder die Impulsfrequenz entsprechend geändert werden. Nach einer relativ kurzen Wartezeit bis zum Erhalt neuer Messwerte kann dann automatisch eine ein- oder mehrfache Feinjustierung von Elektromagnetfeld und/oder Schallwechseldruck ausgeführt werden. Anstelle des Steuergerätes 5 (Computer) kann auch eine Person (Arzt) die entsprechende Nachregulierung durchführen. Ziel ist es dabei, bestimmte Funktionsmerkmale der Mikrozirkulation therapierelevant zu verbessern, d.h. in die Nähe oder auf die Höhe von Normalwerten zu bringen.

Für das Steuergerät 5 sind daher einzelne Regler oder Regelmechanismen für die Frequenz und für die Impulsfolge des elektromagnetischen Feldes, für die elektromagnetische Flussdichte des elektromagnetischen Feldes, für den Frequenzbereich der Teilschwingungen des elastomechanischen Feldes, für die Schallschnelle und den Schallwechseldruck des elastomechanischen Signals vorgesehen.

Durch die erfindungsgemäße Vorrichtung ist eine individuelle Anpassung durch Steuerung der Signalreize möglich, wodurch der Behandlungserfolg deutlich verbessert und optimiert wird.

Die Erfindung betrifft auch ein prophylaktisches oder therapeutisches Verfahren zur Stimulierung der lokalen Regulation der Mikrozirkulation, das dadurch gekennzeichnet ist, dass der Körper oder ein Teil des Körpers eines Patienten einem biorhythmisch definierten elektromagnetischen Feld mit unterschiedlichen Impulsfolgen, Frequenzwerten und Magnetfeldstärken von Signalen ausgesetzt wird und zusätzlich einem elastomechanischen Signal mit Schall-Leistungen im Bereich von $10^{-5}$ W bis 100 W, Schallstärken im Bereich von $10^{-12}$ W/m$^2$ bis 1 W/m$^2$ und Schallwechseldrücken im Bereich von 0,2 • $10^{-4}$ N/m$^2$ bis $10^3$ N/m$^2$,

wobei eine Messwerterfassungeinrichtung die erfassten Werte zu einem Steuergerät leitet und von diesem veränderte Steuerimpulse in Form von elektromagnetischen und elastomechanischen Signalen über einen Impulsgenerator, eine Felderzeugungseinrichtung und parallel über einen Schallgeber zum Körper des Patienten geleitet werden, und dieser Vorgang ein- oder mehrmals wiederholt wird bis zum Erreichen von Zielwerten mikrozirkulatorischer Funktionsmerkmale, und wobei die Messwerterfassungeinrichtung Geräte zur Intravitalmikroskopie und kombinierten Weißlichtspektroskopie und Laser-DOPPLER-Mikroflussmessung umfasst.

Bevorzugt bestehen die Impulsfolgen des elektromagnetischen Feldes aus Grundsignalen und Zusatzsignalen, bei denen das Zusatzsignal eine wenigstens 20 mT höhere magnetische Flussdichte als das Grundsignal hat. Weiterhin ist bevorzugt, dass das elektromagnetische Signal bei den Amplituden der Einzelimpulse einer Exponentialfunktion folgt oder bogenförmig an- und absteigenden Hüllkurven mit harmonischem oder anharmonischem Verlauf.

Die für die Messwerterfassung eingesetzten Geräte zur Intravitalmikroskopie (Hersteller: Institut für Mikrozirkulation, Berlin, DE) und zur kombinierten Weißlichtspektroskopie und Laser-DOPPLER-Mikroflussmessung sind in ihrer Arbeitsweise im Fachgebiet bekannt. Die Intravitalmikroskopie ist ein bildgebendes Verfahren, das Live-Darstellungen in einer ausgewählten oberflächennahen Geweberegion der Mikrogefäßreaktionen (Vasomotionsphänomene) sowie Messungen geometrischer und dynamischer Merkmale in der Mikrozirkulation auf nicht-invasive Weise übermittelt.

Das Gerät zur kombinierten Weißlichtspektroskopie und Laser-DOPPLER-Mikroflussmessung (Hersteller LEA Medizintechnik GmbH, Gießen, Deutschland) gestattet die Ermittlung von mikrozirkulatorischen Meßdaten in zwei Gewebetiefen - ca. 2 mm und 6 mm - für die Merkmale relative Hämoglobinsättigung, venolenseitige Sauerstoffausschöpfung, Strömungsgeschwindigkeiten und Strömungsfluss der roten Blutzellen.

Die Erfindung betrifft auch die Verwendung einer Vorrichtung, umfassend einen Impulsgenerator und eine Felderzeugungseinrichtung, die unterschiedliche Impulsfolgen, Frequenzwerte und Magnetfeldstärken veranlassen, und zusätzlich einen separaten Schallgeber für ein elastomechanisches Signal mit Schall-Leistungen im Bereich von $10^{-5}$ W bis 100 W, Schallstärken im Bereich von $10^{-12}$ W/m$^2$ bis 1 W/m$^2$ und Schallwechseldrücken im Bereich von 0,2 • $10^{-4}$ N/m$^2$ bis $10^3$ N/m$^2$,

zur Erzeugung eines biorhythmisch definierten elastomechanischen Feldes, und Zuführung der über eine Messwerterfassungeinrichtung erhaltenen Messwerte des Patienten zu einem Steuergerät, und von diesem leitet es veränderte Steuerimpulse in Form von elektromagnetischen Signalen über einen Impulsgenerator und eine Felderzeugungseinrichtung und von elastomechanischen Signalen parallel dazu über einen Schallgeber wieder zum Körper eines Patienten.

Die Verwendung der erfindungsgemäßen Vorrichtung führt zur Verbesserung des Funktionszustandes der Mikrozirkulation, was anhand repräsentativer Merkmale der Mikrozirkulation nachweisbar ist. Dies tritt insbesondere auf bei der Prophylaxe, bei Rehabilitanden und bei chronischen Wundheilungsstörungen, sowie bei Diabetes mellitus Typ II und peripheren Durchblutungsstörungen. Die erfindungsgemäße Verwendung ist auch zur Nachsorge nach schulmedizinischer Therapie von Krebsleiden, Erkrankungen des Muskel-Bänder-Skelettsystems, Erkrankungen des rheumatischen Formenkreises und Regenerationsstörungen anwendbar und führt zu bemerkenswerten Verbesserungen auf diesen Gebieten. Das betrifft gesundheitseinschränkende oder krankhafte Zustände, sowohl lokal als auch gesamtorganismisch.

Neben einer deutlichen Steigerung der Merkmalbeträge mikrozirkulatorischer Funktionsmerkmale bei der erfindungsgemäßen Vorrichtung tritt auch eine therapierelevante Verlängerung der Abklingzeiten der Merkmaländerungen auf, und dies bis zu verdoppelten Zeiträumen.

Bestimmt man den Zeitpunkt nach Stimulierung, an dem die höchsten Beiträge der Änderungen mikrozirkulatorischer Funktionsmerkmale auftreten und misst den Zeitpunkt, an dem noch 50% dieser Merkmalbeiträge vorhanden sind, so werden bis zu sechsfach verlängerte Abklingzeiten bei Einwirkung der Kombination von elektromagnetisches Feld und elastomechanisches Feld gegenüber der alleinigen Anwendung eines einzelnen Feldes festgestellt.

Die erfindungsgemäße Vorrichtung kann eingesetzt werden zur Stimulation der lokalen Durchblutungsregulation beim Menschen und verschiedenen Säugetierspezies sowie anderen Arten, bei denen Mechanismen der lokalen Durchblutungsregulation in der Evolution ausgebildet wurden.

Die Anwendung des kombinierten Stimulationssignals aus elektromagnetischem und elastomechanischem Feld dient einer interaktiven Stimulierung der lokalen Regulation der Mikrozirkulation. Simultan erhobene intravitalmikroskopische Befunde sowie reflexionsspektroskopische und Laser-DOPPLER-Mikroflussbefunde zum Funktionszustand der Mikrozirkulation dienen einer interaktiven Festlegung der zu applizierenden Größen des elektromagnetischen und elastomechanischen Feldes zur Herbeiführung oder Optimierung des therapeutischen Erfolges. Diese Interaktion kann durch einen Computer oder einen Arzt erfolgen. Die computergestützte Befundauswertung ermöglicht eine interaktive, an die jeweiligen Gegebenheiten des betreffenden Patienten angepasste Regulationsstimulation der Mikrozirkulation.

Die Erfindung wird nachstehend durch Beispiele näher erläutert. In den dazugehörigen Zeichnungen zeigen

Fig. 1    Schematische Darstellung der Stimulierungsvorrichtung für die lokale Regulation der Mikrozirkulation
Fig. 2    Säulendiagramm der venolenseitigen Sauerstoffausschöpfung $\Delta pO_2$ in der Subkutis
Fig. 3    Säulendiagramm der venolenseitigen Sauerstoffausschöpfung $\Delta pO_2$ in der Wadenmuskulatur
Fig. 4    Säulendiagramm des Strömungsflusses der roten Blutzellen $Q_{RBC}$ in der Subkutis
Fig. 5    Säulendiagramm des Strömungsflusses der roten Blutzellen $Q_{RBC}$ in der Wadenmuskulatur

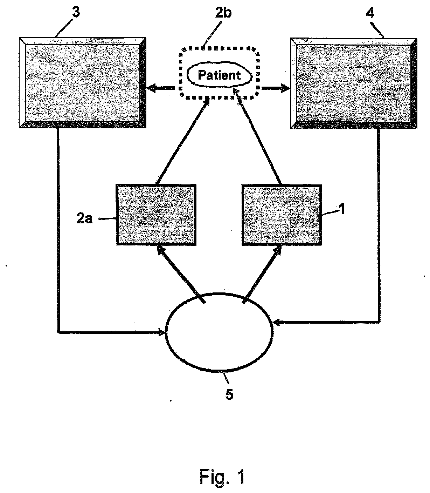

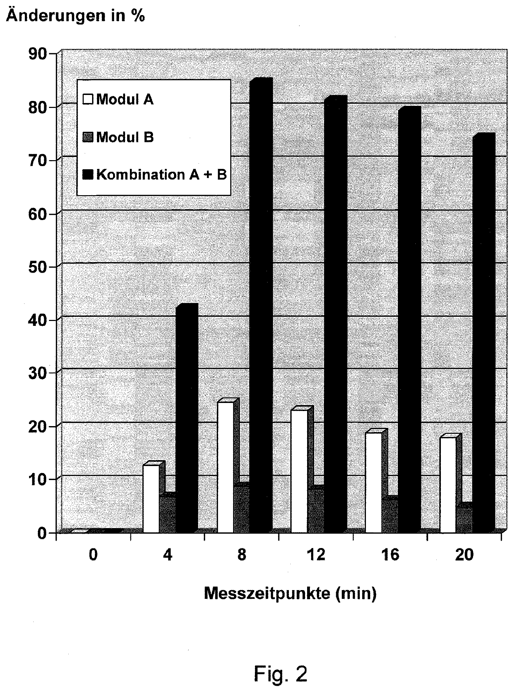

In Fig. 1 ist ein System zur Stimulierung der lokalen Regulation der Mikrozirkulation dargestellt.
Von dem Schallgeber 1 wird ein elastomechanisches Signal direkt über eine Platte, angeordnet auf der Hautoberfläche des Patienten, auf diesen übertragen. Gleichzeitig wird von dem Impulsgenerator 2a ein Impuls auf die Felderzeugungseinrichtung 2b gegeben. Die Felderzeugungseinrichtung 2b ist beispielsweise eine Matte, in der ein bestimmtes elektromagnetisches Feld erzeugt wird und auf der der Patient gelagert ist. In Fig. 2 - 5 sind 2a und 2b auch als "Modul A" bezeichnet und der Schallgeber 1 als "Modul B".
Die bei dem Patienten über das Gerät zur Intravitalmikroskopie 3, bestehend aus Mikroskop, Monitor und Kamerasystem, erfassten Werte, werden dem Steuergerät 5 zugeleitet. Gleichzeitig werden auch die durch die Messerfassungseinrichtung zur kombinierten Weißlichtspektroskopie und Laser-DOPPLER-Mikroflussmessung 4 erhaltenen Werte dem Steuergerät 5 zugeleitet. Der durch diese Werte erhaltene Befund wird durch eine interaktive, an die jeweiligen Gegebenheiten des Patienten angepasste Regulations-stimulation durch einen Computer ausgewertet. Es werden dann neue, geänderte Signale entsprechend dem ersten Ergebnis (Befund) bei dem Patienten an den Schallgeber 1 und den Impulsgenerator 2a und die damit verbundene Felderzeugungseinrichtung 2b ausgesandt, bis der Regelkreis nach mehrmaliger Wiederholung abgeänderter Impulse zu einem angemessenen, therapeutisch relevanten Ergebnis geführt hat.

Beispiel 1

Eine Gruppe ambulanter Rehabilitanden mit Diabetes mellitus Typ II wurde ausgewählt. Gesamtstichprobe N = 18, Alter etwa 60 bis 70 Jahre, Körpermasse etwa 80 kg, Körperlänge etwa 175 cm, wobei der BMI (Body Mass Index) meist im oberen Normbereich lag, zum Teil leichtes Übergewicht.
Es wurden validierte, hochauflösende klinische Messverfahren auf dem neuesten Stand der Technik eingesetzt, wie Weißlichtspektroskopie in Kombination mit Laser-DOPPLER-Mikroflussmessung (System LEA, Gießen, Deutschland).
Erfasst wurden komplette mikrovaskuläre Netzwerke in der Wadenmuskulatur, linke Wade bei einer Eindringtiefe von ~ 3 mm zur Erkennung der Wirkung in den subkutanen Netzwerken. Bei den Mikrogefäßen der Wadenmuskulatur wurden solche mit Diametern $\leq$ 100 $\mu$m erfasst: Arteriolen, Kapillaren, Venolen.
Als gemessene mikrozirkulatorische Funktionsmerkmale wurden ausgewählt

-    die venolenseitige Sauerstoffausschöpfung $\Delta pO_2$ als prozentuale Änderungen im Vergleich mit den Ausgangswerten 0. min, welche gleich 0 gesetzt wurden, und.
-    der Strömungsfluss der roten Blutzellen $Q_{RBC}$, ebenfalls als prozentuale Änderungen im Vergleich mit den Ausgangswerten 0. min, welche gleich 0 gesetzt wurden.

Messzeitpunkte : Äquidistante Messzeitpunkte 0.min (Ausgangswerte), 4.min, 8.min, 12.min, 16.min und 20.min.
Biometrie: WILCOXON-Rangsummentest (alpha = 5%) als parameterfreies Prüfverfahren für kleine Stichproben.
Dauer der Behandlungen 8 min. Einmalige Behandlung. Lokale Applikationen im Bereich der unteren Extremitäten.
Folgende Behandlungsoptionen wurden angewandt:

1 Nur Behandlung mit elektromagnetischem Feld [System BEMER (BEMER classic; Stufe 5) mit einer Impulsfolge und Frequenz entsprechend Beispiel 1, Verumgruppe, gemäß WO 2011/023634] - in den Figuren "Modul A".
2. Nur Behandlung mit einem biorhythmisch definierten Schallfeld (Mittelwerte des Schallwechseldruckes 0,5 • 10$^{-2}$

N/m$^2$ - in den Figuren "Modul B".

3. Gleichzeitige Behandlung gemäß Option 1 und 2 - in den Figuren "Modul A + B"

Die Reihenfolge der Behandlungsoptionen erfolgte per Zufallsgenerator, der zeitliche Abstand der Behandlungsoptionen lag bei 1 bis 2 Tagen.

Gemäß Fig.2 ist die venolenseitige Sauerstoffausschöpfung als prozentuale Änderung im Vergleich mit den Ausgangswerten 0. min, die gleich 0 gesetzt wurden, im Vergleich der Behandlungsoptionen bei der erfindungsgemäßen kombinierten Behandlung auch nach 20 Minuten noch immer fast bei 75% Änderung und damit signifikant größer als bei den Behandlungsoptionen 1 und 2, also nur der elektromagnetischen oder nur mit dem Schallfeld, die zu diesem Zeitpunkt bereits auf 4 und 18 % abgeklungen sind.

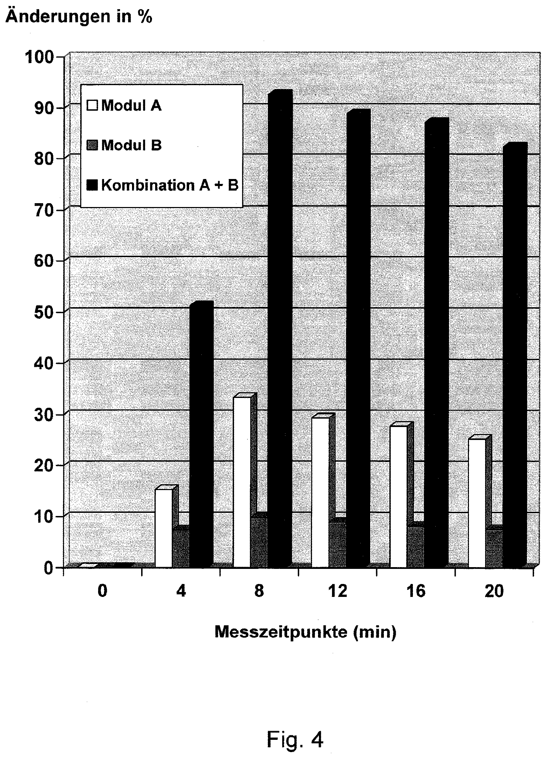

Gemäß Fig.4 ist der Strömungsfluss der roten Blutzellen als prozentuale Änderung im Vergleich mit den Ausgangswerten 0. min, die gleich 0 gesetzt wurden, im Vergleich der Behandlungsoptionen bei der erfindungsgemäßen kombinierten Behandlung auch nach 20 Minuten noch immer bei über 80% Änderung und damit sehr erheblich größer als bei den Behandlungsoptionen 1 und 2, also nur der elektromagnetischen oder nur mit dem Schallfeld, die zu diesem Zeitpunkt bereits zwischen 8 und 25 % liegen.

Bemerkenswert in beiden Diagrammen ist gegenüber der sehr hohen Wirkung die relativ geringe Abklingquote, wodurch nachhaltigere Effekte im Gewebe deutlich werden.

Beispiel 2

Es wurde wie im Beispiel 1 verfahren. Mit der gleichen Gruppe ambulanter Rehabilitanden wie im Beispiel 1 wurde die Wadenmuskulatur den gleichen Behandlungsoptionen unterzogen. Gemessen wurden dabei komplette mikrovaskuläre Netzwerke in der Wadenmuskulatur, linke Wade in einer Eindringtiefe von 6 - 8 mm. Bei den Mikrogefäßen der Wadenmuskulatur wurden solche mit Diametern $\leq 100\ \mu m$ erfasst: Arteriolen, Kapillaren, Venolen.

Die gemessenen Merkmale und die Behandlungsoptionen entsprachen ebenfalls denen des Beispiels 1.

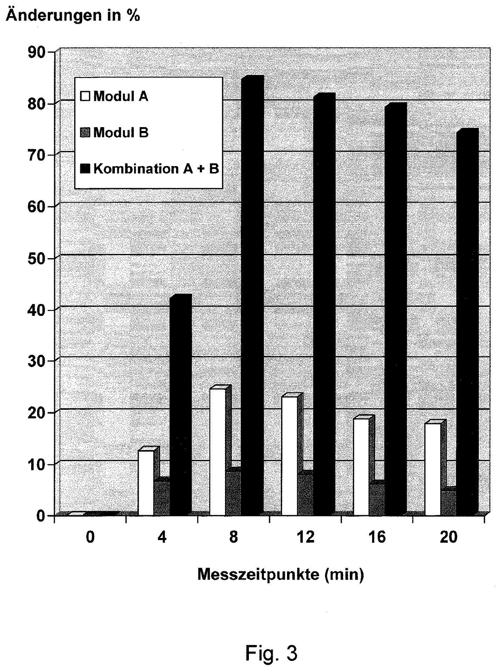

Gemäß Fig.3 ist die venolenseitige Sauerstoffausschöpfung als prozentuale Änderung im Vergleich mit den Ausgangswerten 0. min, die gleich 0 gesetzt wurden, im Vergleich der Behandlungsoptionen bei der erfindungsgemäßen kombinierten Behandlung auch nach 20 Minuten noch immer fast bei 75% Änderung und damit deutlich größer als bei den Behandlungsoptionen 1 und 2, also nur der elektromagnetischen oder nur mit dem Schallfeld, die zu diesem Zeitpunkt zwischen 5 und 18 % liegen.

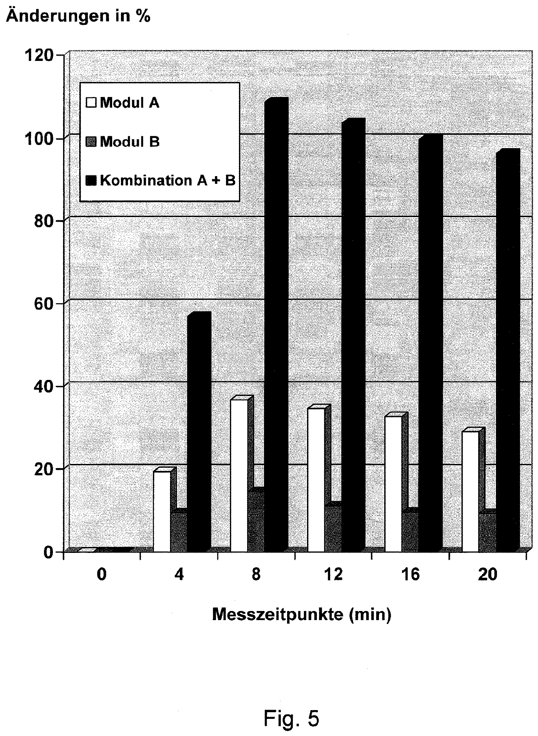

Gemäß Fig.5 ist der Strömungsfluss der roten Blutzellen als prozentuale Änderung im Vergleich mit den Ausgangswerten 0. min, die gleich 0 gesetzt wurden, im Vergleich der Behandlungsoptionen bei der erfindungsgemäßen kombinierten Behandlung auch nach 20 Minuten noch immer bei nahe 100% Änderung und damit erheblich größer als bei den Behandlungsoptionen 1 und 2, also nur der elektromagnetischen oder nur mit dem Schallfeld, die zu diesem Zeitpunkt lediglich zwischen 9 und 28 % liegen.

Auch hier ist bemerkenswert in beiden Diagrammen gegenüber der sehr hohen Wirkung die relativ geringe Abklingquote, wodurch nachhaltigere Effekte im Gewebe deutlich werden.

Beispiel 3

Es wurde wie im Beispiel 1 verfahren. Es erfolgte eine zweimalige Behandlung, beginnend mit den Behandlungsoptionen wie im Beispiel 1. Nach Erhalt der Werte im Steuergerät 5 wurden die Vorgaben für das elektromagnetische Feld geändert (BEMER classic, Stufe 7 und Zusatzsignal) und für das elastomechanische Feld (Wechselschalldruck 10$^{-2}$ N/m$^2$). Für die venolenseitige Sauerstoffausschöpfung ergab sich nach 12 Minuten ein Wert von 90% und nach 20 Minuten ein Wert von 85% Änderung. Dies zeigt, dass insbesondere die Abklingquote bereits nach einmaliger Veränderung der Behandlungsmaßnahmen gegenüber der Erstbehandlung (75% Änderung nach 20 Minuten) signifikant verbessert wurde.

**Patentansprüche**

1. Vorrichtung zur Stimulierung der lokalen Regulation der Mikrozirkulation unter Einbeziehung eines Impulsgenerators (2a) und einer Felderzeugungseinrichtung (2b) zur Erzeugung eines biorhythmisch definierten elektromagnetischen Feldes mit unterschiedlichen Impulsfolgen, Frequenzwerten und Magnetfeldstärken sowie einer Messwerterfassungseinrichtung, umfassend Geräte zur Intravitalmikroskopie (3) und kombinierten Weißlichtspektroskopie und Laser-Doppler-Mikroflussmessung (4) **dadurch gekennzeichnet, dass**
die Vorrichtung einen separaten Schallgeber (1) für ein elastomechanisches Signal zur Erzeugung eines biorhythmisch definierten elastomechanischen Feldes aufweist, wobei der Schallgeber (1) Schall-Leistungen im Bereich von $10^{-5}$ W bis 100 W, Schallstärken im Bereich von $10^{-12}$ W/m$^2$ bis 1 W/m$^2$ und Schallwechseldrücke im Bereich von 0,2 • $10^{-4}$ N/m$^2$ bis $10^3$ N/m$^2$ hat,

und ein Steuergerät (5) zur Koordinierung eingehender Messwerte der Messwerterfassungseinrichtung und ausgehender Steuerimpulse auf den Impulsgenerator (2a), die Felderzeugungseinrichtung (2b) und den Schallgeber (1).

2. Vorrichtung nach Anspruch 1, worin das elastomechanische Signal sinusförmige Teilschwingungen aufweist mit Frequenzen im Bereich von 6 bis 12 Hz und 26 bis 36 Hz.

3. Vorrichtung nach Anspruch 1 oder 2, worin, die sinusförmigen Teilschwingungen 1 bis 5 Wellenberge und dazugehörige Wellentäler pro Minute aufweisen.

4. Vorrichtung nach Anspruch 3, worin die Flanken der Wellenberge oder Wellentäler parabolischen Funktionen entsprechen mit Halbwertsbreiten von 5 bis 10 Sekunden.

5. Vorrichtung nach einem der Ansprüche 1 bis 4, worin das elastomechanische Signal ein Schallspektrum aufweist mit Frequenzen, die in beliebigen verschiedenen Verhältnissen zueinander stehen, oder ein Klangspektrum mit Frequenzen, die ein ganzzahliges Vielfaches eines Grundtones darstellen.

6. Vorrichtung nach einem der Ansprüche 1 bis 5, worin die Schallwechseldrücke im Bereich von 0,5 • $10^{-3}$ N/m$^2$ bis $10^{2}$ N/m$^2$ liegen.

7. Vorrichtung nach einem der Ansprüche 1 bis 6, worin die Flussdichte des elektromagnetischen Feldes im Bereich von 5 $\mu$T bis 0,1 T liegt.

8. Vorrichtung nach einem der Ansprüche 1 bis 7, worin die Flussdichte des elektromagnetischen Feldes im Bereich von 150 $\mu$T bis 90 mT liegt.

9. Vorrichtung nach einem der Ansprüche 1 bis 8, worin der Schallgeber ausgewählt ist aus der Gruppe, bestehend aus Membranen, schwingungsfähigen Platten, sogenannten Schallköpfen, elektromechanischen Wandlern, Thermophonen und mehreren davon.

10. Prophylaktisches oder therapeutisches Verfahren zur Stimulierung der lokalen Regulation der Mikrozirkulation, **dadurch gekennzeichnet, dass** der Körper oder ein Teil des Körpers eines Patienten einem biorhythmisch definierten elektromagnetischen Feld mit unterschiedlichen Impulsfolgen, Frequenzwerten und Magnetfeldstärken von Signalen ausgesetzt wird und zusätzlich einem elastomechanischen Signal mit Schall-Leistungen im Bereich von $10^{-5}$ W bis 100 W, Schallstärken im Bereich von $10^{-12}$ W/m$^2$ bis 1 W/m$^2$ und Schallwechseldrücken im Bereich von 0,2 • $10^{-4}$ N/m$^2$ bis $10^3$ N/m$^2$,
wobei eine Messwerterfassungeinrichtung die erfassten Werte zu einem Steuergerät (5) leitet und von diesem veränderte Steuerimpulse in Form von elektromagnetischen Signalen über einen Impulsgenerator (2a), eine Felderzeugungseinrichtung (2b) und von elastomechanischen Signalen parallel über einen Schallgeber (1) zum Körper des Patienten geleitet werden, und dieser Vorgang ein- oder mehrmals wiederholt wird bis zum Erreichen von Zielwerten mikrozirkulatorischer Funktionsmerkmale, und wobei die Messwerterfassungeinrichtung Geräte zur Intravitalmikroskopie (3) und kombinierten Weißlichtspektroskopie und Laser-DOPPLER-Mikroflussmessung (4) umfasst.

11. Verfahren nach Anspruch 10, worin die Targetstrukturen des elektromagnetischen Feldes und des elastomechanischen Signals mikrovaskuläre Netzwerke im oberflächennahen Gewebe sind.

12. Verwendung einer Vorrichtung, umfassend einen Impulsgenerator und eine Felderzeugungseinrichtung, die unterschiedliche Impulsfolgen, Frequenzwerte und Magnetfeldstärken veranlassen, und zusätzlich einen separaten Schallgeber für ein elastomechanisches Signal mit Schall-Leistungen im Bereich von $10^{-5}$ W bis 100 W, Schallstärken im Bereich von $10^{-12}$ W/m$^2$ bis 1 W/m$^2$ und Schallwechseldrücken im Bereich von 0,2 • $10^{-4}$ N/m$^2$ bis $10^3$ N/m$^2$, zur Erzeugung eines biorhythmisch definierten elastomechanischen Feldes,
und Zuführung der über eine Messwerterfassungeinrichtung erhaltenen Messwerte des Patienten zu einem Steuergerät,
wobei von dem Steuergerät veränderte Steuerimpulse in Form von elektromagnetischen Signalen über den Impulsgenerator und die Felderzeugungseinrichtung und in Form von elastomechanischen Signalen über den Schallgeber zum Körper des Patienten geleitet werden.

**13.** Verwendung nach Anspruch 12 zur Verbesserung repräsentativer Merkmale des Funktionszustandes der Mikrozirkulation bei der Prophylaxe, bei Rehabilitanden und bei chronischen Wundheilungsstörungen, Diabetes mellitus Typ II, peripheren Durchblutungsstörungen, bei Nachsorge nach schulmedizinischer Therapie von Krebsleiden, Erkrankungen des Muskel-Bänder-Skelettsystems, Erkrankungen des rheumatischen Formenkreises und bei Regenerationsstörungen.

Fig. 1

Änderungen in %

Fig. 2

**Änderungen in %**

Fig. 3

Änderungen in %

Fig. 4

**Änderungen in %**

Fig. 5

Europäisches Patentamt
European Patent Office
Office européen des brevets

# EUROPÄISCHER RECHERCHENBERICHT

Nummer der Anmeldung

EP 17 20 8438

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | Anonym: "8 D System (iLifeSOMM)", ILifeSOMM , 21. März 2017 (2017-03-21), Seiten 1-17, XP055453621, Gefunden im Internet: URL:https://web.archive.org/web/2017032116 0534/http://www.ilifesomm.com/8-d-system/ [gefunden am 2018-02-22] * Seite 6 - Seite 10 * * Seite 2 * ----- | 1-13 | INV. A61N1/40 ADD. A61M37/00 A61N1/36 A61N2/00 A61N2/02 A61M21/00 |
| X A | DE 196 15 557 A1 (WELLHAUSEN HANS [DE]) 23. Oktober 1997 (1997-10-23) * Spalte 1, Absatz 2 - Absatz 4; Ansprüche 1-6; Abbildungen 1, 2 * ----- | 1-9 10-13 | |
| A | Anonym: "Produkte (iLifeSOMM)", ILifeSOMM , 10. August 2017 (2017-08-10), Seiten 1-10, XP055453643, Gefunden im Internet: URL:https://web.archive.org/web/2017032116 0534/http://www.ilifesomm.com/ [gefunden am 2018-02-22] * Seite 5 - Seite 8 * ----- -/-- | 1-13 | RECHERCHIERTE SACHGEBIETE (IPC) A61M A61N |

Der vorliegende Recherchenbericht wurde für alle Patentansprüche erstellt

| Recherchenort | Abschlußdatum der Recherche | Prüfer |
|---|---|---|
| München | 26. Februar 2018 | Lorenz, Larissa |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
...........................................................................
& : Mitglied der gleichen Patentfamilie, übereinstimmendes Dokument

EPO FORM 1503 03.82 (P04C03)

Seite 1 von 3

Europäisches
Patentamt

European
Patent Office

Office européen
des brevets

**EUROPÄISCHER RECHERCHENBERICHT**

**Nummer der Anmeldung**

EP 17 20 8438

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | Michael A Lang ET AL: "PROCEEDINGS 1 ST INTERNATIONAL IMIN CONFERENCE", International Microvascular Net , 24. September 2016 (2016-09-24), Seiten 1-69, XP055453661, Brussels Gefunden im Internet: URL:https://www.imin-org.eu/images/science/%202016%20IMIN%20Orlando%20Proceedings.pdf [gefunden am 2018-02-22] * Seite 13 - Seite 29 * ----- | 1-13 | |
| A | Anonym: "Die Frequenzen beim Vibrationstraining", Medicos Vibrationstraining , 4. November 2016 (2016-11-04), Seiten 1-4, XP055453881, Gefunden im Internet: URL:https://web.archive.org/web/20161104124116/https://www.medicos-vibrationstraining.de/informationen-ueber-vibrationstrainer/die-frequenzen.html [gefunden am 2018-02-23] * Seite 2; Tabelle 1 * ----- | 1-9 | RECHERCHIERTE SACHGEBIETE (IPC) |

-/--

Der vorliegende Recherchenbericht wurde für alle Patentansprüche erstellt

| Recherchenort | Abschlußdatum der Recherche | Prüfer |
|---|---|---|
| München | 26. Februar 2018 | Lorenz, Larissa |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.............................................................................
& : Mitglied der gleichen Patentfamilie, übereinstimmendes Dokument

EPO FORM 1503 03.82 (P04C03)

Seite 2 von 3

Europäisches
Patentamt

European
Patent Office

Office européen
des brevets

## EUROPÄISCHER RECHERCHENBERICHT

Nummer der Anmeldung

EP 17 20 8438

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | Kwan, Rachel Lai-Chu et al.: "Pulsed Electromagnetic Field Therapy Promotes Healing and Microcirculation of Chronic Diabetic Foot Ulcers: A Pilot Study", Advances in Skin & Wound Care, Bd. 28, Nr. 5 1. Mai 2015 (2015-05-01), XP002778508, DOI: 10.1097/01.ASW.0000462012.58911.53 Gefunden im Internet: URL:https://journals.lww.com/aswcjournal/Abstract/2015/05000/Pulsed_Electromagnetic_Field_Therapy_Promotes.7.aspx [gefunden am 2018-02-22] * Zusammenfassung * ----- | 1-13 | |
| A | EP 0 995 463 A1 (KAFKA WOLF A PROF [DE]) 26. April 2000 (2000-04-26) * Absatz [0005] - Absatz [0006] * * Absatz [0021] - Absatz [0023] * * Abbildung 1 * ----- | 1-13 | |
| A | EP 2 050 481 A1 (KAFKA WOLF A PROF DR [DE]) 22. April 2009 (2009-04-22) * Absätze [0015], [0016], [0023], [0024], [0040], [0044] * * Abbildungen 1, 2 * ----- | 1-13 | RECHERCHIERTE SACHGEBIETE (IPC) |
| A | RU 2 492 881 C1 (GIL MUTDINOVA LIRA TALGATOVNA [RU]) 20. September 2013 (2013-09-20) * Absätze [0021], [0022], [0026], [0038], [0039], [0045], [0054] * ----- | 1-13 | |

Der vorliegende Recherchenbericht wurde für alle Patentansprüche erstellt

| Recherchenort | Abschlußdatum der Recherche | Prüfer |
|---|---|---|
| München | 26. Februar 2018 | Lorenz, Larissa |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

                                                        
& : Mitglied der gleichen Patentfamilie, übereinstimmendes Dokument

EPO FORM 1503 03.82 (P04C03)

Seite 3 von 3

**ANHANG ZUM EUROPÄISCHEN RECHERCHENBERICHT
ÜBER DIE EUROPÄISCHE PATENTANMELDUNG NR.**

EP 17 20 8438

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

26-02-2018

| Im Recherchenbericht angeführtes Patentdokument | | Datum der Veröffentlichung | Mitglied(er) der Patentfamilie | | Datum der Veröffentlichung |
|---|---|---|---|---|---|
| DE 19615557 | A1 | 23-10-1997 | KEINE | | |
| EP 0995463 | A1 | 26-04-2000 | AT | 204184 T | 15-09-2001 |
| | | | CZ | 9903694 A3 | 16-05-2001 |
| | | | DE | 59801224 D1 | 20-09-2001 |
| | | | DK | 0995463 T3 | 12-11-2001 |
| | | | EP | 0995463 A1 | 26-04-2000 |
| | | | ES | 2161008 T3 | 16-11-2001 |
| | | | HU | 9903645 A2 | 28-08-2000 |
| | | | PL | 336109 A1 | 25-04-2000 |
| | | | PT | 995463 E | 30-11-2001 |
| | | | SI | 0995463 T1 | 31-12-2001 |
| | | | SK | 130799 A3 | 12-09-2000 |
| EP 2050481 | A1 | 22-04-2009 | AT | 512695 T | 15-07-2011 |
| | | | EP | 2050481 A1 | 22-04-2009 |
| RU 2492881 | C1 | 20-09-2013 | | | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0995483 A
- WO 2008025731 A
- WO 2011023635 A
- DE 19834148 A1
- WO 2011023634 A